(19) Europäisches Patentamt
European Patent Office
Office européen des brevets

(11) Publication number: 0 101 004 B2

(12) NEW EUROPEAN PATENT SPECIFICATION

(45) Date of publication of the new patent specification: 16.10.91 Bulletin 91/42

(51) Int. Cl.[5]: C07D 209/08, C07D 307/79

(21) Application number: 83107630.2

(22) Date of filing: 03.08.83

# (54) Process for preparing 4-oxo-4, 5, 6, 7-tetrahydroindole derivative.

The file contains technical information submitted after the application was filed and not included in this specification

(30) Priority: 06.08.82 JP 136232/82

(43) Date of publication of application: 22.02.84 Bulletin 84/08

(45) Publication of the grant of the patent: 21.01.87 Bulletin 87/04

(45) Mention of the opposition decision: 16.10.91 Bulletin 91/42

(84) Designated Contracting States: BE CH DE FR GB IT LI NL SE

(56) References cited:
US-A- 4 119 646

(56) References cited:
JUSTUS LIEBIGS ANNALEN DER CHEMIE, vol. 655, 1962, Weinheim/Bergstrasse, H, STETTER et al., "4-Oxo-4.5.6.7.-tetrahydro-indole und 4-Oxo-1.2.3.4.5.6.7.8-octahydrocarbazole", pages 20-26
CHEMISTRY LETTERS, 1980, S. TORIL et al., "A facile synthesis of 4-hydroxyindole via electrochemical oxidative C-C coupling", pages 1603,1604
Bull. Soc. Chim. France (1971), 4041-4047

(73) Proprietor: SAGAMI CHEMICAL RESEARCH CENTER
4-5, Marunouchi 1-chome
Chiyoda-ku Tokyo 100 (JP)

(72) Inventor: Matsumoto, Masakatsu
1-9-2, Minamidai
Sagamihara-shi Kanagawa-ken (JP)
Inventor: Watanabe, Nobuko
937-130, Tokiwa
Kamakura-shi Kanagawa-ken (JP)

(74) Representative: Türk, Dietmar, Dr. rer. nat. et al
Türk, Gille + Hrabal Patentanwälte
Brucknerstrasse 20
W-4000 Düsseldorf 13 (DE)

EP 0 101 004 B2

## Description

The present invention relates to a process for preparing 4-oxo-4,5,6,7-tetrahydroindole derivatives. More specifically, the present invention relates to a process for preparing 4-oxo-4,5,6,7-tetrahydroindole derivatives of the formula (I):

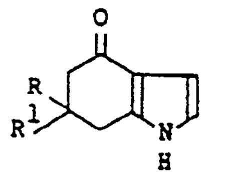

(I)

wherein R and $R^1$ are hydrogen or an alkyl group from a 1,3-cyclohexanedione derivative.

The indole derivatives of the formula (I) are useful not only as a synthetic precursor of 4-hydroxy-indole which is an intermediate for preparing Pindolol being an excellent drug in prevention and treatment of arrhythmia (K. Saemeli: Helv. Physiol. Acta, *25* 221 (1967)), but also as an intermediate for preparing various kinds of 4-substituted indole drugs.

As a method for preparing 4-oxo-4,5,6,7-tetrahydroindole derivatives, there hitherto has been known the following methods such as (i) a method in which 1,3-cyclohexanedione derivative is condensed with a bromopyruvic acid ester in the presence of a base to give a 4(5H)-benzofuranone-3-carboxylic acid, and the furan-ring thereof is converted into a pyrrole ring by treating with ammonia while decarboxylating (H. Stetter and R. Lauterbach; Ann. Chem., *655*, 20(1962)), (ii) a method in which a 1,3-cyclohexanedione derivative is condensed with aminoacetoaldehyde dimethylacetal in the presence of an acid catalyst (J.M. Bobbit and C.P. Dutta: Chem. Comm., 1429(1968)), (iii) a method in which a 1,3-cyclohexanedione derivative and vinyl ether are electrolytically oxidized, and the resulting product is treated with ammonium carbonate (S. Torii and K. Uneyama: Chem. Lett., 1603(1980)), (iv) a method in which 4-(2-pyrrolyl)-butyric acid is subjected to ring closure reaction (M. Julia: Fr. *1540*, 484(1968): Chem. Abstr., *71*, 81163W (1969) and (v) a method in which 4-oxo-4,5,6,7-tetrahydroindole derivatives, optionally substituted by alkyl in 6-position, are reacted with ammonia.

It is an object of the present invention to provide a process for preparing 4-oxo-4,5,6,7-tetrahydroindole derivatives, in good efficiency by using materials easily and inexpensively obtainable.

According to the present invention there is provided a process for preparing a 4-oxo-3,4,5,6-tetrahydroindole derivative of the formula (I):

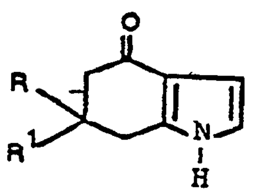

(I)

wherein R and $R^1$ are hydrogen or an alkyl group, from a 1,3-cyclohexanedione derivative which is characterised by

1. reacting a 1,3-cyclohexanedione derivative of formula (III):

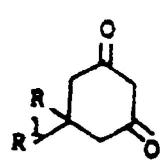

(III)

wherein R and $R^1$ are as defined above, with chloroacetaldehyde in the presence of a base while maintaining at a pH of from 4 to 10, and then treating the reaction mixture with an acid, and

2. reacting the resultant 4-oxo-4,5,6,7-tetrahydrobenzofuran derivative of the formula (II):

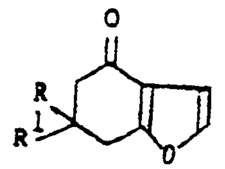

(II)

wherein R and $R^1$ are as defined above with ammonia in water, a combination solvent of water and an alcohol, a combination solvent of water and an etherr, or a combination solvent of water and dimethylformamide.

Preferably the reaction 1) is carried out by

a) mixing a 1,3-cyclohexanedione derivative of formula (III):

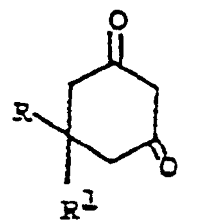

(III)

wherein R and $R^1$ are as defined above, and chloroacetaldehyde in the presence of a base, said base being selected from the group consisting of triethylamine, sodium hydrogencarbonate, potassium carbonate and sodium hydroxide, and being employed in from 1 to 1.2 equimolar amount to the used 1,3-cyclohexanedione derivative,

b) maintaining the reaction mixture at a pH of from 5.4 to 10 thoughout the reaction,

c) after the completion of the reaction, dehydrating the compound(IV) formed

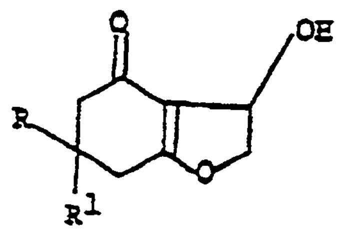

(IV)

by acidifying the resultant reaction mixture to obtain a 4-oxo-4,5,6,7-tetrahydrobenzofuran derivative of formula (II):

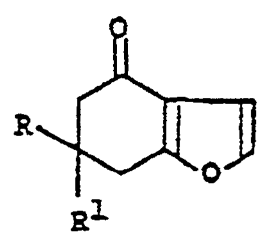

(II)

wherein R and $R^1$ are defined above.

The process step of preparing the final product (I) is an analogous method to that disclosed in Justus Liebigs Annalen der Chemie Vol. 655 (1962) page 20, and the technical feature which characterises this invention lies in the preparation step of the tetrahydrobenzofuran derivative of formula (II).

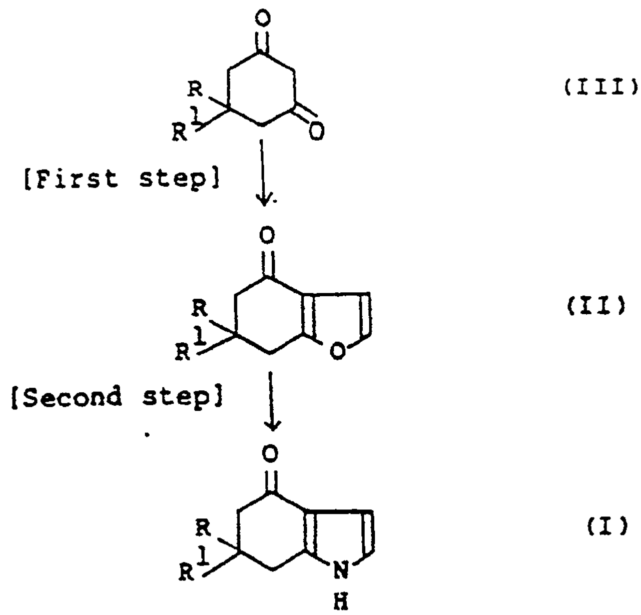


wherein R and $R^1$ are hydrogen or an alkyl group.

[First Step]

In this step, 1,3-cyclohexanedione derivative of the formula (III) is reacted with chloroacetaldehyde, and then the reaction mixture is treated with an acid to give a 4-oxo-4,5,6,7-tetrahydrobenzofuran derivative of the formula (II).

The above 1,3-cyclohexanedione derivatives and chloroacetaldehyde can be easily obtained by industrial processes.

In this step, it is important to maintain the reaction mixture at a pH of from 4 to 10 in order to adjust the balance between the desired reaction and the dissociation equilibrium between the monomer and polymer of chloroacetaldehyde, since chloroacetaldehyde is hard to exist in the reaction mixture as a monomer. The reaction efficiency is poor at a pH of less than 4 and more than 10.

The process of the first step is essentially carried out in the presence of a base. Examples of the preferred base are sodium hydroxide, potassium carbonate, sodium hydrogencarbonate, and the organic bases such as triethylamine. The base is generally employed in an equimolar amount of slightly excess amount to the used 1,3-cyclohexanedione derivative of formula (II).

Though the process of the first step is preferably carried out in water for smoothly proceeding the reaction, and organic solvent such as ethyl acetate, dichloromethane or toluene can be used in combination with water when the reaction materials and the product are slightly soluble in water. The use of an alcoholic solvent such as methanol having a nucleophilicity is not suitable because such a solvent causes a side reaction. The reaction is easily proceeded at a temperature of from -20°C to 100°C, preferably from -10°C to a room temperature for producing the desired compound in good efficiency.

After completing the above reaction, it is necessary to treat the reaction mixture with an acid for dehydrating the following compound (IV), because the reaction is terminated at the following step under the above reaction conditions:

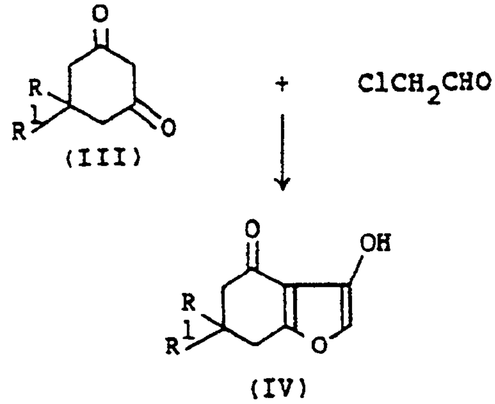


(III)

(IV)

Examples of the acid are, for instance, inorganic acids such as sulfuric acid hydrochloric acid, organic acids such as sulfonic acid, which are usually utilized in the dehydration reaction. The reaction can be carried out without specially heating.

[Second Step]

In this step oxo-4,5,6,7-tetrahydrobenzofuran derivative of the formula (II) obtained in the first step is reacted with ammonia to give a 4-oxo-4,5,6,7-tetrahydroindole derivative of the formula (I).

As the ammonia used in the second step, any source of ammonia such as aqueous ammonia, ammonia gas or ammonium carbonate can be used.

The reaction of the second step is carried out in a solvent which does not affect the reaction, i.e. in water or a combination solvent of water and an alcohol such as methanol, ethanol or propanol, a combination solvent of water and an ether such as tetrahydrofuran, dioxane, dimethoxyethane, diethylene glycol or dimethyl ether, or a combination solvent of water and dimethyl formamide.

The reaction is carried out at room temperature of from 100°C to 200°C, preferably from 130°C to 170°C for producing the desired compound in good efficiency.

The present invention is more specifically described and explained by means of the following Examples and comparative Example, in which all % are by weight unless otherwise noted. It is to be understood that the present invention is not limited to the Examples, and various changes and modifications may be made in the invention without departing from the spirit and scope thereof.

Examples 1 to 13

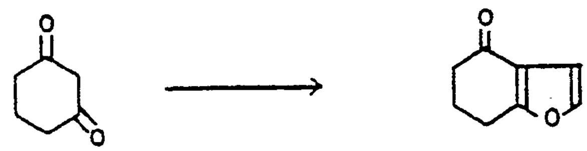

To 5 ml of water were added 1.12 g (10 mmoles) of 1,3-cyclohexanedione and the each base shown in Table 1, and then 2 ml of 40% aqueous solution of chloroacetoaldehyde was added thereto. The resulting mixture was stirred at room temperature for 2 hours. To the reaction mixture was added about 10 ml of ethyl acetate, and then the reaction mixture was acidified with 0.5 to 1 ml of sulfuric acid and stirred for 30 minutes. The resulting ethyl acetate layer was analyzed by gas-liquid chromatography (hereinafter referred to as "GLC") (SE30 10% 1.2 m ×3 mmϕ glass column, 130°C) using p-dimethoxybenzene as an internal standard. The obtained each result is shown in Table 1. It was proved from the results of the GLC that 4-oxo-4,5,6,7-tetrahydrobenzofuran was produced in a yield shown in Table 1, in each Example.

TABLE 1

pH of the reaction mixture

| Ex. | Base (mmole) | Start | End | Yield (%) |
|---|---|---|---|---|
| 1 | none Comparative example | <1 | <1 | 7 |
| 2 | NaOH (5) | 1.4 | 0.9 | 26 |
| 3 | $CaCO_3$ (6) | 4.3 | 5.9 | 36 |
| 4 | $CH_3CO_2Na$ (12) | 4.4 | 4.2 | 41 |
| 5 | Pyridine (12) | 4.5 | 4.8 | 44 |
| 6 | Triethylamine (12) | 5.4 | 5.5 | 58 |
| 7 | $NaHCO_3$ (12) | 5.8 | 6.2 | 61 |
| 8 | $Ba(OH)_2$ (6) | 6.0 | 6.2 | 48 |
| 9 | NaOH (10) | 7.6 | 7.6 | 66 |
| 10 | NaOH★ | 8.2 | 7.7 | 64 |
| 11 | NaOH★ | 10 | 8.9 | 56 |
| 12 | $Ca(OH)_2$ (10) | 11.6 | 10.8 | 30 |
| 13 | NaOH★ | 12 | 9.8 | 19 |

★ The pH was adjusted with 2N NaOH solution.

Example 14

To a mixture of 1.12 g (10 mmoles) of 1,3-cyclohexanedione and 8 ml of water was added 1.38 g (10 mmoles) of potassium carbonate, and then 2 ml of 40% aqueous solution of chloroacetoaldehyde was added thereto. The resulting mixture was stirred at room temperature for 45 hours. The reaction mixture was maintened at a pH of from 7.75 to 9.50 throughout the reaction. After completing the reaction, the obtained reaction mixture was treated and analyzed in the same manner as in Example 1. It was proved from the results of the analysis that 4-oxo-4,5,6,7-tetrahydrobenzofuran was produced in a 68% yield.

Example 15

To a mixture of 80 ml of water and 20 ml of 40% aqueous solution of chloroacetaldehyde was added 10.0 g (119 mmoles) of sodium hydrogencarbonate while cooling with ice. To the resulting reaction mixture was added dropwise 90 ml of aqueous solution containing 11.2 g (100 mmoles) of 1,3-cyclohexanedione while cooling with ice at a rate of 0.4 ml/minute. The reaction mixture was maintained at a pH of from 9 to 6 throughout the reaction. After completing the dropping, the reaction mixture was stirred at a room temperature overnight. To the resulting reaction mixture was added about 100 ml of ethyl acetate, and then the reaction mixture was acidified (pH<1) with sulfuric acid and stirred for about one hour. The ethyl acetate layer was separated, washed with an aqueous solution of potassium carbonate, and dried on magnesium sulfate. After distilling away the ethyl acetate, the obtained residue was distilled under a reduced pressure to give 10.3 g (yield: 76%) of 4-oxo-4,5,6.7-tetrahydrobenzofuran being a colorless oily material (boiling point: 66°C/133 Pa (1 torr).

Example 16

To a mixture of 10 ml of ethyl acetate, 2 ml of 40% aqueous solution of chloroacetaldehyde and about 3 ml of water was added 1.0 g (12 mmoles) of sodium hydrogencarbonate while cooling with ice, and then the resulting mixture was stirred. To the reaction mixture was added dropwise 5 ml of aqueous solution containing 1.12 g of 1,3-cyclohexanedione while cooling with ice at a rate of 0.05 ml/minute. The reaction mixture was maintained at a pH of from 6.2 to 8.7 throughout the reaction. After completing the dropping, the reaction mixture was stirred at room temperature overnight, and then acidified with sulfuric acid. After stirring the mixture for one hour, the ethyl acetate layer was separated, washed with an aqueous solution of potassium carbonate for removing the unreacted 1,3-cyclohexanedione, and then dried on magnesium sulfate. After distilling away the solvent, the residue was applied to a column of silica gel and eluted with dichloromethane to give 1.09 g (yield: 80%) of 4-oxo-4,5,6,7-tetrahydrobenzofuran.

Example 17

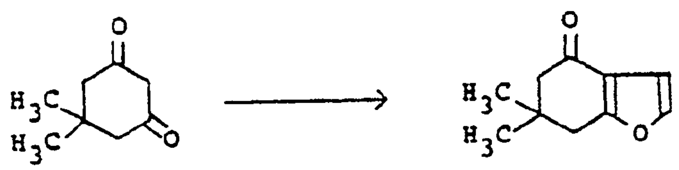

To a mixture of 24 ml of water and 6 ml of 40% aqueous solution of chloroacetaldehyde was added 0.7 g (8 mmoles) of sodium hydrogencarbonate while cooling with ice. To the reaction mixture were added dropwise 4.2 g (30 mmoles) of 5,5-dimethyl-1,3-cyclohexanedione and 40 ml of aqueous solution containing 2.3 g (27 mmoles) of sodium hydrogencarbonate while cooling with ice at a rate of 0.4 ml/minute. The reaction mixture was maintained at a pH of from 5.7 to 8.0 throughout the reaction. After completing the dropping, the reaction mixture was stirred at a room temperature overnight, and then treated in the same manner as in Example 1 to give 2.36 g (yield: 48.2%) of 4-oxo-6,6,-dimethyl-4,5,6,7-tetrahydrobenzofuran being a colorless oily material (boiling point: 78°C/0.93 Pa (0.7 mmHg).

Example 18

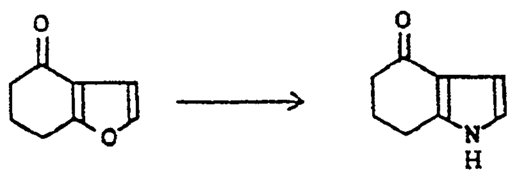

To a mixture of 5 ml of ethanol and 5ml of 29% aqueous ammonia was added 1.0 g 4-oxo-4,5,6,7-tetrahydrofuran, the mixture was stirred in a sealed tube at 150°C for 3 hours. After completing the reaction and distilling away ammonia and water, the reaction mixture was dissolved in 25 ml of hot water and filtered. After cooling the filtrate, the precipitated flake crystal was gathered and dried to give 4-oxo-4,5,6,7-tetrahydroindole in a yield of 60%

Example 19

The procedure of Example 18 was repeated except that 3 ml of ethanol and 7 ml of water were used instead of 5 ml of ethanol and 5 ml of water, and the reaction mixture was subjected to reaction for about 12 hours instead of 3 hours. The resulting reaction mixture was concentrated, and the resulting residue was applied to a column of silica gel and eluted with a mixed solvent of acetone and ethyl acetate to give 950 mg (yield: 96%) of 4-oxo-4,5,6,7-tetrahydroindole.

Example 20

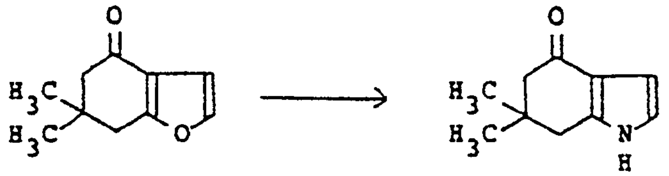

To a mixture of 2 ml ethanol and 7 ml of 29% aqueous ammonia was added 1.0 g 6,6-dimethyl-4-oxo-4,5,6,7-tetrahydrobenzofuran, and the reaction mixture was stirred in a sealed tube at 130°C to 140°C for about 12 hours. After concentrating the reaction mixture, the resulting residue was applied to a column of silica gel and eluted with dichloromethane, and then ethyl acetate to give 300 mg of the starting material, 6,6-dimethyl-4-oxo-4,5,6,7-tetrahydrobenzofuran and 160 mg of the desired 6,6-dimethyl-4-oxo-4,5,6,7-tetrahydroindole.

## Claims

1. A process for preparing a 4-oxo-4,5,6,7-tetrahydroindole derivative of formula (I):

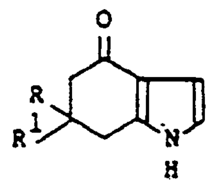

(I)

wherein R and $R^1$ are hydrogen or an alkyl group, from a 1,3-cyclohexanedione derivative, characterised by

1) reacting a 1,3-cyclohexanedione derivative of formula (III):

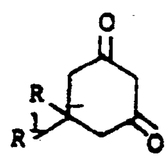

(III)

wherein R and $R^1$ are as defined above, with chloroacetaldehyde in the presence of a base while maintaining at a pH of from 4 to 10, and then treating the reaction mixture with an acid, and

2) reacting the resultant 4-oxo-4,5,6,7-tetrahydrobenzofuran derivative of the formula (II):

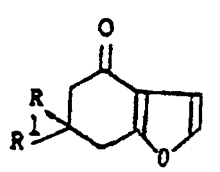

(II)

wherein R and $R^1$ are as defined above, with ammonia in water, a combination solvent of water and an alcohol, a combination solvent of water and an ether, or a combination solvent of water and dimethylformamide.

2. A process of claim 1, wherein the reaction 1) is characterised by

a) mixing a 1,3-cyclohexanedione derivative of formula (III):

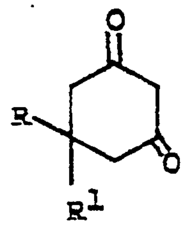

(III)

wherein R and R[1] are as defined above, and chloroacetaldehyde in the presence of a base, said base being selected from the group consisting of triethylamine, sodium hydrogencarbonate, potassium carbonate and sodium hydroxide, and being employed in from 1 to 1.2 equimolar amount to the used 1,3-cyclohexanedione derivative,

b) maintaining the reaction mixture at a pH of from 5.4 to 10 thoughout the reaction, and

c) after the completion of the reaction, dehydrating the compond (IV) formed

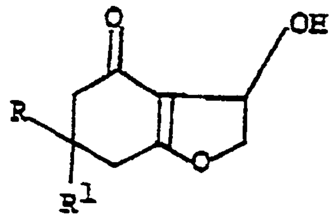

(IV)

by acidifying the resultant reaction mixture to obtain a 4-oxo-4,5,6,7-tetrahydrobenzofuran derivative of formula (II):

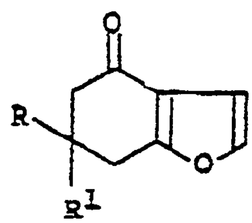

(II)

wherein R and R[1] are defined above.

## Patentansprüche

1. Verfahren zur Herstellung eines 4-Oxo-4,5,6,7-tetrahydroindolderivats der Formel (I):

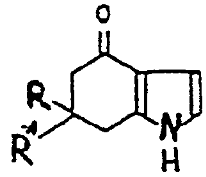

(I),

worin R und R[1] Wasserstoff oder eine Alkylgruppe sind, aus einem 1,3-Cyclohexandionderivat, gekennzeichnet durch

1) Reaktion eines 1,3-Cyclohexandionderivats der Formel (III):

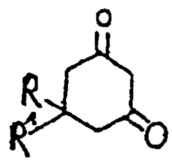

(III),

worin R und $R^1$ jeweils wie oben definiert sind, mit Chloracetaldehyd in Gegenwart einer Base, wobei ein pH-Wert von 4 bis 10 eingehalten wird, und dann Behandeln der Reaktionsmischung mit einer Säure und
2) Reaktion des resultierenden 4-Oxo-4,5,6,7-tetrahydrobenzofuranderivats der Formel (II):

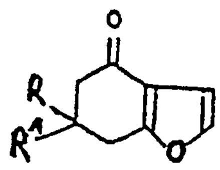

(II),

worin R und $R^1$ wie oben definiert sind, mit Ammoniak in Wasser, einer Lösungsmittelkombination von Wasser und einem Alkohol, einer Lösungsmittelkombination von Wasser und einem Äther oder einer Lösungsmittelkombination von Wasser und Dimethylformamid.

2. Verfahren nach Anspruch 1, worin die Reaktion 1) gekennzeichnet ist durch

a) Mischen eines 1,3-Cyclohexandionderivats der Formel (III):

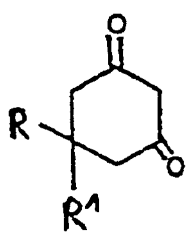

(III),

worin R und $R^1$ wie oben definiert sind, und Chloracetaldehyd in , Gegenwart einer Base, die aus der Gruppe, die aus Triethylamin, Natriumhydrogencarbonat, Kaliumcarbonat und Natriumhydroxyd besteht, ausgewählt ist und in einer 1 bis 1,2 äquimolaren Menge zum eingesetzten 1,3-Cyclohexandionderivats verwendet wird,

b) Halten der Reaktionsmischung bei einem pH-Wert von 5,4 bis 10 während der Reaktion, und

c) nach Vervollständigen der Reaktion, Dehydratisierung der entstandenen Verbindung

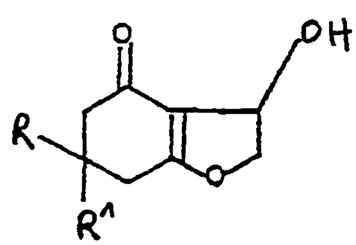

(IV)

durch Ansäuern der resultierenden Reaktionsmischung, um ein 4-Oxo-4,5,6,7-tetrahydrobenzofuranderivat der Formel (II) zu erhalten:

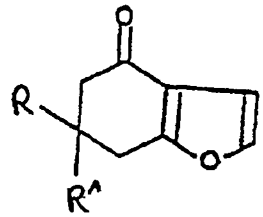

(II),

worin R und $R^1$ wie oben definiert sind.

## Revendications

1. Procédé pour préparer un dérivé 4-oxo-4,5,6,7-tetrahydroindole de formule (I) :

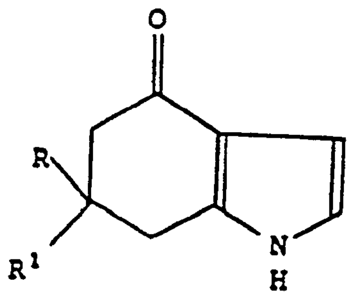

(I)

dans laquelle R et $R^1$ sont l'atome d'hydrogène ou un groupe alkyle, à partir d'un dérivé 1,3-cyclohexanedione, caractérisé en ce que

1) on fait réagir un dérivé de 1,3-cyclohexanedione de formule (III) :

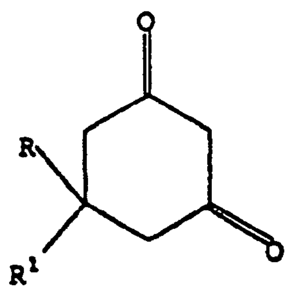

(III)

dans laquelle R et $R^1$ sont comme définis ci-dessus, avec du chloroacétaldéhyde en présence d'une base tout en maintenant le pH entre 4 et 10, et ensuite en ce que l'on traite le milieu réactionnel avec un acide, et

2) en ce que l'on fait réagir le dérivé 4-oxo-4,5,6,7-tetrahydrobenzofurane résultant de formule (II) :

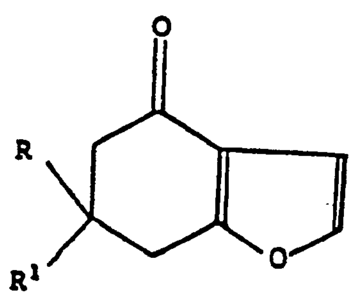

(II)

dans laquelle R et $R^1$ sont comme définis ci-dessus, en présence d'ammoniac aqueux, d'une combinaison

solvatante d'eau et d'un alcool, d'une combinaison solvatante d'eau et d'un éther, ou d'une combinaison solvatante d'eau et de diméthylformamide.

2. Procédé selon la revendication 1, dans laquelle la réaction 1) est caractérisé en ce que :

a) on mélange un dérivé de 1,3-cyclohexanedione de formule (III) :

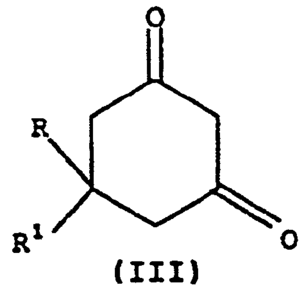

(III)

dans laquelle R et $R^1$ sont comme définis ci-dessus et du chloroacétaldéhyde en présence d'une base, ladite base étant choisie parmi le groupe formé par la triéthylamine, le bicarbonate de sodium, le carbonate de potassium et la soude, et en ce qu'il est employé dans une quantité allant de 1 à 1,2 molaire par rapport au dérivé 1,3-cyclohexanedione utilisé,

b) en ce qu'on maintient le milieu réactionnel à un pH allant de 5,4 à 10 tout au long de la réaction et,

c) après achèvement de la réaction, en ce que l'on déshydrate le composé (IV) formé :

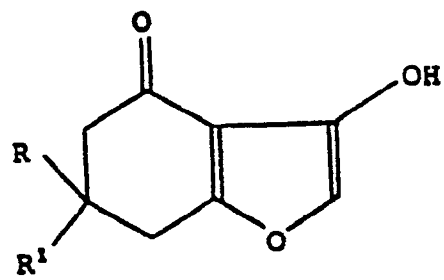

(IV)

par acidification du mélange réactionnel résultant afin d'obtenir un dérivé 4-oxo-4,5,6,7-tétrahydrobenzofurane de formule (II) :

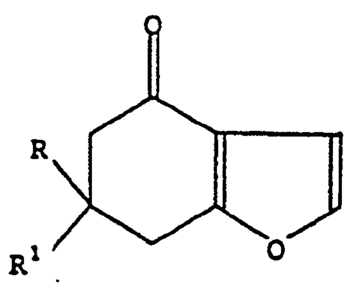

(II)

dans laquelle R et $R^1$ sont comme définis ci-dessus.